# EUROPEAN PATENT APPLICATION

(11) **EP 1 897 544 A1**
(43) Date of publication of application: **12.03.2008**
(21) Application number: 06120103.4
(22) Date of filing: 05.09.2006
(51) Int. Cl.: A61K 31/485, A61K 9/00, A61K 9/50, A61K 9/16

(54) **Opioid agonist and antagonist combinations**

(71) Applicant: Hermann, Holger Lars, 8834 Schindellegi (CH); Hammer, Kerstin, 8834 Schindellegi (CH)
(72) Inventor: Hermann, Holger Lars, 8834 Schindellegi (CH); Hammer, Kerstin, 8834 Schindellegi (CH)
(74) Representative: Heinemann, Monica

(57) **Abstract**

A composition comprising (a) an opioid agonist in non-retarded form, (b) an opioid agonist in retarded form and encapsulated in an enteric coating, (c) an opioid antagonist in non-retarded form and (d) an opioid antagonist in retarded form and encapsulated in an enteric coating, wherein components (a) and (b), and (c) and (d) may not be present in the same particle. The composition may be used for the manufacture of a medicament for treatment of pain and/or substitution therapy of opioid addiction and/or detoxification of opioids.

## Description

The subject invention relates to a composition comprising an opioid agonist in non-retarded form, an opioid agonist in retarded form and encapsulated by an enteric coating, an opioid antagonist in non-retarded form and an opioid antagonist in retarded form and encapsulated by an enteric coating.

WO 01/58447 describes the combination of opioid agonists and opioid antagonists for the treatment of inter alia analgesia, hyperexcitability and physical dependence. The products are provided in the form of a tablet and can contain a core material which contains the agonist and the antagonist in retarded form and within an enteric coating. On top of this coating the unprotected or non-retarded agonist and antagonist can be coated and the whole mixture is provided in the form of a tablet.

Such formulations have the disadvantage that the release of the unprotected agonist and antagonist only starts in the stomach. Moreover, not all of the unprotected agonist and antagonist is absorbed within the stomach which in turn can lead to uncontrolled resolution and absorption of the core material in the gastro-intestinal tract. This is in particular a disadvantage for the treatment of opioid addicts who need an immediate response if the concentration of e.g. heroin is decreased. This has the further disadvantage that opioid side effects such as nausea, vomiting and mouth dryness are not immediately influenced. Thus, the object of the subject invention is to overcome these disadvantages.

This can be achieved by a composition comprising
(a) an opioid agonist in non-retarded form,
(b) an opioid agonist in retarded form and encapsulated in an enteric coating,
(c) an opioid antagonist in non-retarded form and
(d) an opioid antagonist in retarded form and encapsulated in an enteric coating,
wherein components (a) and (b), and (c) and (d) may not be present in the same particle.

Thus, possible combinations of the components (a) to (d) are:
- (a), (b), (c) and (d) are each present in a separate particle.
- The next possibility is that components (a) and (c) are combined in one separate particle and components (b) and (d) are combined in another separate particle.
- A further combination would be that components (a) and (c) are combined in one separate particle and components (b) and (d) are each present in a separate particle.
- The last combination would thus be that components (a) and (c) are each present in a separate particle and components (b) and (d) are combined in one particle.

The advantage of the composition according to the invention is that the dissolution and resorption of the active materials can start immediately. The present invention utilizes thereby the different resorption behavior of the non-retarded and retarded components in the different resorption areas in the human body. A patient's first resorption area would be the mouth with its mucosa with a pH of about 7. The next region is the oesophagus with a pH of 7 (upper region) to 1 (lower region). The next areas are the stomach with a pH of 1 to 2.5, the small intestine with a pH of 6 to 7, appendix with a pH of 7.5, and finally colon and rectum with a pH of 6.7 to 7.

Thus, the inventive composition is poured and dissolved into e.g. water and taken orally by the patient. The dissolved materials, i.e. in particular the non-retarded and unprotected active ingredients can be absorbed by the mucosa of the mouth and by the oesophagus during swallowing. This is in particular true for all the opioid antagonists. The preferred opioid antagonist naloxone has the effect that side effects which often occur in conjunction with misuse of opioids such as extensive sweating, vomiting, nausea or xerostomia (dry mouth) can be avoided or at least decreased. In contrast, this is not possible with the tablet according to WO 01/58447 which is not absorbed in the mouth and the oesophagus so that it does not have any influence on these side effects. Moreover, the opioid antagonist in the tablets of WO 01/58447 which is absorbed in the stomach and in the gastro-intestinal tract is metabolized in the liver by the first-pass effect with an antagonist with low bio-availability like naloxone and has then no further activity. However, the opioid antagonist as used in accordance with the present invention and in particular naloxone which is absorbed via the mouth mucosa and the oesophagus is directly transported into the brain and is resorbed partly sublingually, transported also through the Vena cava superior without liver filtrance, and can have its pharmaceutical activity without any metabolism and degradation.

In addition, the preferred opioid antagonists, in particular naloxone, show only a low systemic bio-availability when applied according to the invention. "Low" means less than 10 % oral bio-availability, preferably less than 5 % oral bio-availability, more preferably less than 4 % and most preferably 0.1 to 3 % as defined by the WHO Annex 9, 1996, as the rate and extent of availability of an active drug ingredient from a dosage form as determined by its concentration-time curve in the systemic circulation or by its excretion in the urine. The bio-availability can only be determined in relation to a different application form. Generally, it is compared with intravenous application wherein the bio-availability is defined as 100 %. A decrease in bio-availability can occur due to metabolism of the substance in the liver within the so-called first-pass effect. The present invention utilizes the low systemic bio-availability of Naloxone at a local accumulation before the first-pass effect occurs.

Within the meaning of this invention, an agonist is a substance which is able to stimulate the receptor (e.g. µ, kappa and/or delta receptors) and which has a pharmacological effect. Antagonists in contrast can also bind to the same receptors (e.g. µ, kappa and/or delta opioid receptors) but do not show a pharmacological effect. In general, it can be said that antagonists block the receptor.

The term "opioid" is well known in the art and comprises narcotic analgesics (opiates and opioids).

In accordance with the present invention, it is preferred that at least the non-retarded components (a) and (c) are in the form of a powder, more preferably the retarded and non-retarded components (a) to (d) are in form of a powder. The components (a) to (d) of the composition can also be in the form of beats, pearls, globules, spherules or pellets. The average particle size of these materials ranges from 200 microns to 2 mm, preferably from 280 microns to 1.2 mm and more preferably from 300 microns to 1 mm. It is further possible that the particle size of components (a) to (d) may be within the same range or a different range. The particle size may be determined by common methods including light scattering (also elastic-laser-light-scattering), optical means, sedimentation or other means known in the art. Another possibility would be the determination of the particle size via dry seaving or the coulter-counter analysis. The method used in the Example of this invention is dry seaving (ASTM D 1921).

Any common agonist known in the art can be used for the composition of the present invention. Preferably, the agonist is selected from the group comprising morphine, codeine, fentanyl, fentanyl analogs, heroin, hydromorphone, oxycodone, hydrocodone, pentacozine, buprenorphine, methadone, enkephalins, dynorphins, similarly acting alkaloids and opioid peptides and pharmaceutically acceptable salts, esters, ethers and hydrates thereof. Moreover, mixtures of different agonists may be used. As opioid antagonists, common agonists known in the art can be used in the subject invention. Preferably, the opioid antagonist is selected from the group comprising naloxone, naltrexone, nalmefene, nalide, nalnexone, nalorphine, nalpuphine, nalorphine, dinicotinate, and pharmaceutically acceptable salts, esters, ethers and hydrates thereof. Moreover, mixtures of different antagonists may be used. Most preferably, the opioid agonist is morphine or a pharmaceutically acceptable salt, ester, ether or hydrate thereof. As opioid antagonist, naloxone and a pharmaceutically acceptable salt, ester, ether and/or hydrate thereof is used.

Pharmaceutically acceptable salts are preferably selected from chlorine, bromine, iodine, sulphate, phosphate, tartrate, acetate, mucate, succinate, phthalate and citrate. Pharmaceutically acceptable hydrates are preferably selected from mono-, di-, tri-, tetra- and pentahydrates. It is also possible that tautomeric forms or tautomeric derivatives of the active ingredients are used where such a tautomeric form is existent. The composition according to the invention can further comprise pharmaceutically acceptable carriers as well as other excipients. Suitable carriers are inter alia lactose, kaolin, saccharose, talcum, gelatine, agar, pectine, magnesium stearate and stearic acid. Suitable excipients are e.g. diluents, lubricants, binders, granulating aids, colorants, flavorants, glidants that are conventional in the pharmaceutical art. The composition according to the invention may further comprise one or more other active ingredients, in particular active ingredients for pain therapy, antidepressants, tranquilizers etc. Such additional active agent may be included in the particles forming the inventive composition as described above or introduced in sachets as described below, in particular in particulate form such as a powder.

The ratio of opioid agonist (e.g. morphine) to opioid antagonist (e.g. naloxone) is from 100 : 1 to 100 : 25, i.e. for example 100 mg morphine and 1-25 mg naloxone. Preferably, the ratio of opioid agonist to opioid antagonist is from 100 : 1 to 100 : 5. An addicted person needs a daily dose of about 300-800 mg/day (about 4 to 10 mg/kg body weight) whereas a person suffering from pain needs a daily dose of about 100-400 mg/day (about 1 to 5 mg/kg body weight).

The opioid agonist in retarded form and the opioid antagonist in retarded form are encapsulated in an enteric coating (enteric material). An enteric coating is in general a material which delays the release of a medication until the dosage form has passed through the acidic medium of the stomach (pH 1-5). The definition of an enteric coating or enteric material can be derived from the US pharmacopeia (USP) (USP 23, chapter <724> discloses that the material is tested with e.g. the "paddle method" at 50 rpm at 37 °C; an enteric coating has to withstand 2 hours in 0.1 M HCl with a maximum release of active ingredient of less than 10 %). The material for the enteric coating is in general a polymer, e.g. cellulose and derivatives, methacrylic acid copolymers, hydroxypropyl methylcellulose (HPMC), hydroxypropyl methylcellulose phthalate and hydroxypropyl methylcellulose acetate succinate. Different types of methacrylic acid copolymers can be used. They include methacrylic acid copolymer type A (Eudragit^{®} L-100), methacrylic acid copolymer type B (Eudragit^{®} S-100), methacrylic acid copolymer type C (Eudragit^{®} L 30 D 55, Eudragit^{®} L-100-55), a polymer of methacrylic acid methyl metacrylate and methyl metacrylate (Eudragit^{®} FS) and mixtures thereof. The enteric layer may further comprise other agents such as cellulose acetate phthalate, polyvinyl acetate phthalate, cellulose acetate trimellitate, shellac and/or zein. Optionally, the enteric layer further comprises anti-tackiness agents such as talc or glyceryl monostearate; plasticizers such as triethylcitrate or polyethylene glycol; and pigments such as titanium dioxide or ferric oxides. The enteric layer may further comprise one or more plasticizers including acetyl triethyl citrate, acetyltributyl citrate, acetylated monoglycerides, glycerin, triacetin, propylene glycol, phthalate esters (e.g., diethyl phthalate, dibutyl phthalate), castor oil, sorbitol and dibutyl seccate.

A retarded form, also known as sustained-release or slow-release formulation, is well known to those skilled in the art. In a retarded application form, the active ingredient is embedded in a matrix from which the active ingredient is released slowly and constantly over a defined period of time. Preferred materials for the retarded forms are cellulose derivatives, in particular ethyl cellulose or hydroxypropyl methyl cellulose (HPMC). However, any retarded or "slow-release" formulation known in the art may be used in the present invention. Most preferred retard formulations are methacrylic acid ester copolymers with e.g. COOCH₃ or COOC₄H₉ groups which are commercial available from Degussa (Röhm Pharma) under the tradename Eudragit^{®} NE 30 D/40 D, or ammonium alkyl methacrylate copolymers which have functional groups with quaternary ammonium (trimethyl ammonium ethyl methacrylate) e.g. COOCH₂-CH₂N⁺(CH₃)₃Cl⁻. Such components are commercially available as Eudragit^{®} RL 100/RS 100 granulate, Eudragit^{®} RL PO/RS PO solid, Eudragit^{®} RL 30 D/RS 30 D dispersion (30%) or Eudragit^{®} RL 12,5/RS 12,5 organic solution (12.5%). Both the methacrylic acid ester copolymers and the ammonium alkyl metacrylate copolymers are insoluble, permeable and pH-independent and are able to release the substance over a specified period of time.

The composition of the present invention can be used for the manufacture of a medicament for the treatment of pain and/or substitution therapy of opioid addiction and/or detoxification of opioids.

Dependency or addiction is generally defined as various forms of being in need of certain substances, attitudes or behaviour. The range reaches thereby from simple habits to dependency with enormous destructive potential. "Various forms" means physical dependence and psychological dependence. The main criterias for physical dependency are:
- development of tolerance, which means that the subjected person has to increase the dose of a substance to achieve the same effect. The result is that the doses increase more and more;
- if the drug is discontinued, physical withdrawal symptoms appear. These withdrawal symptoms are substance-specific;
- substances are consumed in order to avoid or alleviate the withdrawal symptoms. The symptoms within psychological dependence are an intense and sometimes overwhelming need of consuming a substance with the aim of generating a positive perception or avoiding a negative perception. The affected person has only a diminished control on the termination of consume as well as on the amount of substance being used.

The treatment of pain and/or substitution therapy with the composition according to the invention has the advantage that a therapeutically effective level is maintained relatively constant for 24 hours while side effects are reduced. A therapeutically effective level means the level wherein an individual subjected to the composition experiences a desired effect with a minimum of side effects. This therapeutic effective level is normally achieved when a therapeutically effective amount is administered which, however, varies within different individuals. This therapeutically effective amount is administered to the person in need thereof in form of a medicament. As the Plexi myentericii which are topically situated around the Gl tract are relevant for the constipation and other side effects and are treated by the antagonist before liver filtrance, side effects are blocked.

Thus, another object of the invention is to provide a medicament which comprises the composition of the present invention.

Most preferably, the medicament according to the invention is in the form of a sachet. Sachet means an oral application form which is powdery but can also be in form of beads, pearls, globules, spherules or pellets. A sachet is administered usually after dissolution/dispersing in a solvent, preferably water.

An advantage of the sachet of the invention is that, as discussed in the foregoing, the non-retarded opioid antagonist effectively reduces side effects, and that the non-retarded opioid agonist, in particular morphine, is immediately set free. Thus, a patient who is suffering from addiction will experience an immediate effect which is again not possible with the tablets according to WO 01/58447 as those take longer for dissolution and resorption. Such effect achieved in accordance with the present invention is extremely necessary for pain and addiction patients.

After these first effects of the sachet of the invention, i.e. the dissolution and absorption of the non-retarded opioid agonist and antagonist, the remaining ingredients namely the encapsulated opioid agonist and antagonist will pass the stomach and enter the duodenum and intestinal tract. During the relatively long duration of the medicament in the stomach, duodenum and intestinal tract, the enteric coating is degraded and the agonist and antagonist are set free. This liberation is further extended because of the slow release formulation of components (b) and (d). Thus, in total, a long-acting effect lasting about 24 hours starting with an early onset of the activity can be achieved while simultaneously avoiding side effects and maintaining a continuous therapeutic level. At the same time, also the side effect of immobility of the intestinal tract (obstipation) can be avoided because of the continued presence of the opioid antagonist. In addition to the above discussed effects of effectively reducing side effects, withdrawal symptoms and pain on a short and long term basis, the composition of the present invention furthermore leads to a significant reduction of agonist and antagonist level fluctuations (blood, serum levels), resulting in an enhanced protection from acquired tolerance and other side effects.

Another embodiment of the invention concerns a process for the manufacture of a medicament, wherein the process comprises the steps:
- providing an opioid agonist and opioid antagonist in non-retarded form,
- providing an enteric coated and retarded opioid agonist and an enteric coated and retarded antagonist,
- mixing the opioid agonist and opioid antagonist in non-retarded form and the enteric coated and retarded opioid agonist and enteric coated and retarded antagonist and optionally any pharmaceutically acceptable carriers and/or excipients.

The mixing of the components can be performed by any method known in the art, including mixing them together in a screw extruder, mill, fluid bed, and conventional mixers.

The retarded component can be made by dispersing the ingredients preferably in a liquid polymer dispersion (e.g. HPMC) and subjecting the dispersion to wet granulation, followed by extrusion and spheronisation.

The enteric coating can be applied to the opioid agonist and to the opioid antagonist in any conventional manner. For example the enteric coating can be applied to the components in a fluidized bed coating apparatus or via film coating.

As the agonists and antagonists usable in this invention are already known from the prior art, we refer to methods known in the art for preparation of these compounds. In particular, we refer to the "Merck Index (German Edition), 13^{th} edition, entry 6388 "Naloxone" as well as the references cited therein and entry 6300 "Morphine" and the references cited therein. In addition, not yet discovered compounds may be used in accordance with the present invention.

The medicament prepared in accordance with the present invention has the further advantage that it is non-abusable since an intravenous misuse causes distinct aversive effects and withdrawal symptoms.

In the following, the present invention is further illustrated by the way of non limiting examples.

### Examples:

### Example 1. Preparation of the sachet:

1,000 g morphine is dispersed in 3,000 g HPMC and is wet granulated. The wet granulated product is extruded through a 300 micron sieve. The extruded product is subjected to spheronisation to obtain small, round particles with a size of about 300 microns (determined by dry sieving according to ASTM D 1921). The particles are then coated in a fluidized bed coating apparatus in a first step with a protection layer of HPMC. The HPMC coated particles are then coated with Eudragit® S 100 methacrylic acid copolymer in the same apparatus. The same procedure as above is performed with naloxone to obtain naloxone particles with a size of about 300 microns.

The non-retarded morphine and non-retarded naloxone are only coated with a thin HPMC protection layer which has a good solubility and both non-retarded components are also obtained as particles with a size of about 300 microns.

All components are then combined in the appropriate amount to a sachet containing 100 mg of the inventive composition with a morphine:naloxone ratio of 100:5.

### Example 2. Substitution:

A patient with the diagnosis heroin dependency (according to ICD 10 F 11.2) with repeatedly acute opiate intoxications and multiple detoxification attempts and in addition with two long-term attempts of withdrawal of the drug is treated with the composition according to the invention.

The current substitution therapy with methadone shows significant side effects (QT prolongation measured via electrocardiography, obstipation with enormous psychological strain and nausea after methadone intake). The patient also admitted intravenous misuse of the administered substitution medication during the substitution period and admitted additional consume of 2 grams of heroin up to two times a week.

After discontinuation of the methadone program with a final dose of 120 milligram per day of methadone, a change to the new medicament is carried out within 24 hours. The substitution is accepted well. The change is carried out with 600 mg of the new composition which has been taken in a single daily dose in the morning and in a fasting state. The composition is administered in form of a sachet which is introduced in a glass of water and dissolved therein. After 4 weeks flushing-out period of methadone, no evidence of QT prolongation is visible via electrocardiography (ECG). The patient reports normal defecation and reduced nausea.

After additional 4 weeks, a normalization of the patient's sexual behavior occurs which results in an additional social stabilization. The patient reports an attempt of intravenous misuse of the new medication which, however, was stopped because of an aversive effect and distinct withdrawal symptoms (sweating, tremor and lacrimation) due to the intravenous application of the new medication. No further attempt of intravenous misuse was reported. Moreover, a reduction of an additional consume of heroin (which frequently occurs under methadone substitution) was seen. This is due to a reduction of craving for heroin during the substitution therapy with the new medication. The patient can be stably adjusted to 600 milligram per day of the new medication.

### Example 3. Withdrawal:

A patient with the diagnosis of heroin dependency (according to ICD 10 11.2), noticed in 1998, is treated with the new medication for withdrawal of the drug. The patient had multiple intoxications, multiple detoxification attempts and relapsed two times after a long term withdrawal attempt. A new attempt of detoxification after continuous consume of heroin (presently up to 3 gram heroin) is carried out with the new medication.

The patient is first adjusted to 600 milligram per day of the new medication with a daily reduction of 100 milligrams per day and a stabilizing phase of 1 week after the beginning of the study. The administration of the new medication takes place in the morning in a fasting state wherein the appropriate amount of pouches of sachet (one pouch contains 100 milligram sachet) is dissolved in a glass of water and taken by the patient. During the reduction phase, no or only minor withdrawal symptoms can be detected. The patient can be released from ambulant monitoring after 14 days.

### Example 4. Pain:

The treatment of pain is carried out on a female patient with the diagnosis of mammary carcinoma according to ICD 10 C 50.8 (mammary gland, overlapping areas). After surgery and repeated chemotherapy, the patient shows strong or strongest pain (according to WHO a level of 3) which requires the administration of strong opioid analgesics and supporting measures.

The patient has previously been treated with one drop of Tramal per 1 milligram/kg bodyweight (weight of patient: 55 kg). A daily dose of 60 milligram Tramal in the form of drops led to strong obstipation and nausea of the patient. The defecation was only possible with enema and laxatives.

Administering of Tramal is discontinued and the change to the new medication is carried out within 24 hours. The new medication is administered once a day in a dose of 100 milligram in the form of a sachet (1 pouch contains 100 milligram) which is dissolved in a glass of water and taken in the morning in a fasting state. After one week of the medication, the dose is increased to 200 milligram/day.

The new medication results in a clear reduction of pain within a subjective scale of pain. After two weeks of the medication, normalization of defecation with no more need of enemas or intake of laxatives can be monitored. In addition, a clear reduction of nausea occurs with the new medication.

## Claims

1. A composition comprising
(a) an opioid agonist in non-retarded form,
(b) an opioid agonist in retarded form and encapsulated in an enteric coating,
(c) an opioid antagonist in non-retarded form and
(d) an opioid antagonist in retarded form and encapsulated in an enteric coating,
wherein components (a) and (b), and (c) and (d) may not be present in the same particle.

2. The composition according to claim 1, wherein at least the non-retarded components (a) and (c) are in the form of a powder or wherein all components (a) to (d) are in the form of a powder.

3. The composition according to claim 1, wherein components (a) to (d) are in the form of beads, pearls, globules, spherules or pellets.

4. The composition of claims 2 and 3 wherein the particle size of the powder, particles, beads, pearls, globules, spherules or pellets ranges from 200 microns to 2 mm, and wherein the particle size of components (a) to (d) may be within the same range or different ranges.

5. The composition according to any of claims 1 to 4, wherein the opioid agonist is selected from the group comprising morphine, codeine, fentanyl, fentanyl analogs, heroin, hydromorphone, oxycodone, hydrocodone, pentacozine, buprenorphine, methadone, enkephalins, dynorphins, similarly acting alkaloids and opioid peptides and pharmaceutically acceptable salts, esters, ethers and hydrates thereof, and wherein the opioid antagonist is selected from the group comprising naloxone, naltrexone, nalmefene, nalide, nalnexone, nalorphine, nalpuphine, nalorphine, dinicotinate, and pharmaceutically acceptable salts, esters, ethers and hydrates thereof.

6. The composition according to claim 5, wherein the opioid agonist is morphine and pharmaceutically acceptable salts, esters, ethers and hydrates thereof and the opioid antagonist is naloxone and pharmaceutically acceptable salts, esters, ethers and hydrates thereof.

7. The composition of any of claims 1 to 6, wherein the opioid antagonist has a bio-availability (according to WHO Annex 9, 1996) of less than 10 % compared with intravenous application.

8. Use of the composition of any of claims 1 to 7 for the manufacture of a medicament for treatment of pain and/or substitution therapy of opioid addiction and/or detoxification of opioids.

9. The use of claim 8, wherein the composition provides a therapeutically effective level for 24 hours.

10. A medicament comprising the composition of any of claims 1 to 7.

11. The medicament of claim 10 in the form of a sachet.

12. A process for the manufacture of a medicament according to claims 10 and 11 comprising:
• providing an opioid agonist and opioid antagonist in non-retarded form,
• providing an enteric coated and retarded opioid agonist and an enteric coated and retarded antagonist,
• mixing the opioid agonist and opioid antagonist in non-retarded form and the enteric coated and retarded opioid agonist and enteric coated and retarded antagonist and optionally any pharmaceutically acceptable carriers and/or excipients.
